(19) **Europäisches Patentamt**

**European Patent Office**

**Office européen des brevets**

(11) **EP 1 186 584 B1**

(12) **EUROPEAN PATENT SPECIFICATION**

(45) Date of publication and mention
of the grant of the patent:
**21.07.2004 Bulletin 2004/30**

(51) Int Cl.[7]: **C07C 5/27**

(21) Application number: **00307691.6**

(22) Date of filing: **06.09.2000**

(54) **Process for the isomerisation of xylenes with simultaneous conversion of ethylbenzene**

Verfahren zur Isomerisierung von Xylolen mit gleichzeitiger Umsetzung von Ethylbenzol

Procédé pour l'isomérisation du xylène avec conversion simultanée de l'éthylbenzène

(84) Designated Contracting States:
**DE ES FR GB NL**

(43) Date of publication of application:
**13.03.2002 Bulletin 2002/11**

(73) Proprietor: **UOP LLC**
**Des Plaines, IL 60017 (US)**

(72) Inventors:
• **Sharma, Sanjay B.**
**Des Plaines, Illinois 60017 (US)**
• **Riley, Benjamin D.**
**Des Plaines, Illinois 60017 (US)**
• **Gurevich, Sergey V.**
**Des Plaines, Illinois 60017 (US)**
• **Rosinski, Greg A.**
**Des Plaines, Illinois 60017 (US)**

(74) Representative: **Eddowes, Simon et al**
**Urquhart-Dykes & Lord LLP**
**30 Welbeck Street**
**London, W1G 8ER (GB)**

(56) References cited:
**WO-A-98/05613**     **US-A- 4 899 010**

**Description**

This invention relates to catalytic hydrocarbon conversion, and more specifically a process for aromatics isomerization.

BACKGROUND

The xylenes, para-xylene, meta-xylene and ortho-xylene, are important intermediates which find wide and varied application in chemical syntheses. Para-xylene upon oxidation yields terephthalic acid which is used in the manufacture of synthetic textile fibers and resins. Meta-xylene is used in the manufacture of plasticizers, azo dyes, wood preservers, etc. Ortho-xylene is feedstock for phthalic anhydride production.

Xylene isomers from catalytic reforming or other sources generally do not match demand proportions as chemical intermediates, and further comprise ethylbenzene which is difficult to separate or to convert. Para-xylene in particular is a major chemical intermediate with rapidly growing demand, but amounts to only 20-25% of a typical $C_8$-aromatics stream. Adjustment of isomer ratio to demand can be effected by combining xylene-isomer recovery, such as adsorption for para-xylene recovery, with isomerization to yield an additional quantity of the desired isomer. Isomerization converts a non-equilibrium mixture of the xylene isomers which is lean in the desired xylene isomer to a mixture which approaches equilibrium concentrations.

Various catalysts and processes have been developed to effect xylene isomerization. In selecting appropriate technology, it is desirable to run the isomerization process as close to equilibrium as practical in order to maximize the para-xylene yield; however, associated with this is a greater cyclic $C_8$ loss due to side reactions. The approach to equilibrium that is used is an optimized compromise between high $C_8$ cyclic loss at high conversion (i.e. very close approach to equilibrium) and high utility costs due to the large recycle rate of unconverted $C_8$ aromatics.

Catalysts for isomerization of $C_8$ aromatics ordinarily are classified by the manner of processing ethylbenzene associated with the xylene isomers. Ethylbenzene is not easily isomerized to xylenes, but it normally is converted in the isomerization unit because separation from the xylenes by superfractionation or adsorption is very expensive. One approach is to react the ethylbenzene to form a xylene mixture via conversion to and reconversion from naphthenes in the presence of a solid acid catalyst with a hydrogenation-dehydrogenation function. An alternative widely used approach is to dealkylate ethylbenzene to form principally benzene while isomerizing xylenes to a near-equilibrium mixture. The former approach enhances xylene yield by forming xylenes from ethylbenzene, while the latter approach commonly results in higher ethylbenzene conversion, thus lowering the quantity of recycle to the para-xylene recovery unit and concomitant processing costs.

In the past twenty years or so, crystalline aluminosilicate zeolite-containing catalysts have become prominent for xylene isomerization. US-A-3,856,872, for example, teaches xylene isomerization and ethylbenzene conversion with a catalyst containing ZSM-5, -12, or -21 zeolite. US-A-4,626,609 discloses conversion of xylene isomers using a catalyst comprising a composite which has been steamed at 200° to 500°C. US-A-4,899,012 teaches the use of a catalyst containing lead, a Group VIII metal, a pentasil zeolite and an inorganic-oxide binder to isomerize xylenes and dealkylate ethylbenzene. US Patent. No 4 899 010 discloses a process for isomerization of unextracted, ethylbenzene-containing xylene feeds using a platinum containing silicate molecular sieve catalyst. Development efforts continue toward realizing economically attractive isomerization catalysts with a superior combination of activity, selectivity and stability.

SUMMARY

A principal object of the present invention is to provide a novel isomerization process for alkylaromatic hydrocarbons. More specifically, this invention is directed to the processing of $C_8$ aromatics to increase the concentration of a desired xylene isomer.

This invention is based on the discovery that improved conversion of $C_8$ aromatics and selectivity for xylene isomerization can be obtained using a zeolitic catalyst component in a finished catalyst composition that has a certain low water capacity and includes an aluminum phosphate binder in the finished composition.

Accordingly, a broad embodiment of the invention is directed toward an alkylaromatics-isomerization process using a catalyst comprising a zeolitic aluminosilicate having a pore diameter of from 5 to 8Å, a platinum-group metal component and an aluminum phosphate binder, the catalyst being in the form of oil dropped spheres. The process comprises isomerization of a feedstock comprising a non-equilibrium mixture of xylenes and ethylbenzene at isomerization conditions to obtain a product having an increased para-xylene content relative to that of the feedstock. Relevant isomerization conditions comprise a temperature of from 100° to 600°C, a pressure of from 100 kPa to 10 MPa, and a mass hourly space velocity of from 0.5 to 100 hr$^{-1}$. Operation at a temperature of between 350° to 500°C at a mass hourly space velocity of from 10 to 50 hr$^{-1}$ is especially favored. MFI-type zeolite is the favored zeolitic aluminosilicate.

The catalyst has a low $6 \cdot 13 \times 10^2$ Pa (4.6 torr) water capacity of less than 7 mass-%, and preferably between 3 and 5 mass-%. The platinum-group metal preferably comprises platinum in a low concentration of between 100 and 2000 mass-ppm, and optimally between about 200 and 800 mass-ppm, of the catalyst.

These, as well as other objects and embodiments, will become evident from the following detailed description of the invention.

BRIEF DESCRIPTION OF THE DRAWINGS

Figure 1 shows relative activity and selectivity, in terms of process xylenes losses, of catalysts of the invention and the known art.

Figure 2 shows process xylenes losses as a function of catalyst water capacity.

DETAILED DESCRIPTION OF THE INVENTION

The present invention relates to the isomerization of a $C_8$-aromatic mixture containing ethylbenzene and xylenes. Generally such a mixture will have an ethylbenzene content in the approximate range of 5 to 50 mass-%, an ortho-xylene content in the approximate range of 0 to 35 mass-%, a meta-xylene content in the approximate range of 20 to 95 mass-% and a para-xylene content in the approximate range of 0 to 15 mass-%. The feed $C_8$ aromatics comprise a non-equilibrium mixture, i.e., at least one $C_9$-aromatic isomer is present in a concentration that differs substantially from the equilibrium concentration at isomerization conditions. Usually the non-equilibrium mixture is prepared by removal of para- and/or ortho-xylene from a fresh $C_8$-aromatics feed obtained from processes, such as catalytic reforming and/or extraction, for the production and recovery of aromatics from other hydrocarbons.

The alkylaromatic hydrocarbons may be utilized in the present invention as found in appropriate fractions from various refinery petroleum streams, e.g., as individual components or as certain boiling-range fractions obtained by the selective fractionation and distillation of catalytically cracked or reformed hydrocarbons. The isomerizable aromatic hydrocarbons need not be concentrated; the process of this invention allows the isomerization of alkylaromatic-containing streams such as catalytic reformate with or without subsequent aromatics extraction to produce specified xylene isomers and particularly to produce para-xylene. A $C_8$-aromatics feed to the present process may contain nonaromatic hydrocarbons, i.e., naphthenes and paraffins, in an amount up to 30 mass-%. Preferably the isomerizable hydrocarbons consist essentially of aromatics, however, to ensure pure products from downstream recovery processes.

According to the process of the present invention, an alkylaromatic hydrocarbon feed mixture, preferably in admixture with hydrogen, is contacted with a catalyst of the type hereinafter described in an alkylaromatic hydrocarbon isomerization zone. Contacting may be effected using the catalyst in a fixed-bed system, a moving-bed system, a fluidized-bed system, or in a batch-type operation. In view of the danger of attrition loss of the valuable catalyst and of the simpler operation, it is preferred to use a fixed-bed system. In this system, a hydrogen-rich gas and the feed mixture are preheated by suitable heating means to the desired reaction temperature and then passed into an isomerization zone containing a fixed bed of catalyst. The conversion zone may be one or more separate reactors with suitable means therebetween to ensure that the desired isomerization temperature is maintained at the entrance to each zone. The reactants may be contacted with the catalyst bed in either upward-, downward-, or radial-flow fashion, and the reactants may be in the liquid phase, a mixed liquid-vapor phase, or a vapor phase when contacted with the catalyst.

The alkylaromatic feed mixture, which is a non-equilibrium mixture of $C_8$ aromatics, is contacted with the isomerization catalyst at suitable alkylaromatic-isomerization conditions. Such conditions comprise a temperature ranging from 100° to 600°C, and preferably is in the range of from 350° to 500°C. The pressure is from 100 kPa to 10 MPa, and more usually no more than 5 MPa. Sufficient catalyst is contained in the isomerization zone to provide a mass hourly space velocity with respect to the hydrocarbon feed mixture of from 0.5 to 100 hr$^{-1}$, and preferably 2 to 50 hr$^{-1}$; favorable results have been obtained at mass hourly space velocities of at least about 10 hr$^{-1}$ and higher. The hydrocarbon feed mixture optimally is reacted in admixture with hydrogen at a hydrogen/hydrocarbon mole ratio of about 0.5:1 to about 10:1 or more; other inert diluents such as nitrogen, argon and light hydrocarbons may be present.

The particular scheme employed to recover an isomerized product from the effluent of the reactors of the isomerization zone is not deemed to be critical to the instant invention, and any effective recovery scheme known in the art may be used.

In a separation/isomerization process combination relating to the processing of an ethylbenzene/xylene mixture, a fresh $C_8$-aromatics feed is combined with isomerized product comprising $C_8$ aromatics and naphthenes from the isomerization reaction zone and fed to a para-xylene separation zone from which pure para-xylene is recovered. The para-xylene-depleted stream from the separation zone, comprising a non-equilibrium mixture of $C_8$ aromatics, comprising xylenes and ethylbenzene, is fed to the isomerization reaction zone, where the $C_8$-aromatic isomers are

isomerized to near-equilibrium levels to obtain the isomerized product. In this process scheme non-recovered $C_8$-aromatic isomers preferably are recycled to extinction until they are either converted to para-xylene or lost due to side-reactions. Ortho-xylene separation, preferably by fractionation, also may be effected on the fresh $C_8$-aromatic feed or isomerized product, or both in combination, preferably prior to para-xylene separation.

The isomerization catalyst of the present invention comprises zeolitic alumino-silicates having a pore diameter of 5 to 8 Angstroms (Å) preferably selected from those which have a $Si:Al_2$ ratio greater than about 10, preferably greater than 20. Specific examples of suitable zeolites are the MFI, MEL, EUO, FER, MFS, MTT, MTW, TON, MOR and FAU types of zeolites. Pentasil zeolites MFI, MEL, MTW and TON are preferred, and MFI-type zeolites, often designated ZSM-5, are especially preferred.

The catalyst has a $6.3 \times 10^2 Pa$ (4.6 torr) water capacity of less than 7 mass-%.

The relative proportion of zeolite in the catalyst may range from about 10 to about 99 mass-%, with about 20 to about 90 mass-% being preferred. There is a tradeoff between the zeolite content of the catalyst composite and the pressure, temperature and space velocity of an isomerization operation in maintaining low xylene losses.

A refractory binder or matrix is utilized to facilitate fabrication of the isomerization catalyst. The binder should be uniform in composition and relatively refractory to the conditions used in the process.

The binder is an amorphous aluminum phosphate. The zeolite and aluminum phosphate binder may be formed into particles by means well known in the art. A preferred method of preparing the zeolite/aluminum phosphate support involves adding the zeolite either to an alumina sol or a phosphorus compound, forming a mixture of the alumina sol/zeolite/phosphorus compound into particles by employing the oil-drop method described hereinbelow, and calcining the spherical particles.

The preferred oil-drop method of preparing the aluminum phosphate bound catalyst is described in US-A-4,629,717. The technique described in the '717 patent involves the gellation of a hydrosol of alumina which contains a phosphorus compound using the well-known oil-drop method. Generally this technique involves preparing a hydrosol by digesting aluminum in aqueous hydrochloric acid at reflux temperatures of about 80° to 105°C. The ratio of aluminum to chloride in the sol ranges from about 0.7:1 to 1.5:1 mass ratio. A phosphorus compound is now added to the sol. Preferred phosphorus compounds are phosphoric acid, phosphorous acid and ammonium phosphate. The relative amount of phosphorus and aluminum expressed in molar ratios ranges from about 10:1 to 1:100, respectively, on an elemental basis. The zeolite is added to the aluminum phosphate hydrosol and the mixture is gelled. One method of gelling this mixture involves combining a gelling agent with the mixture and then dispersing the resultant combined mixture into an oil bath or tower which has been heated to elevated temperatures such that gellation occurs with the formation of spheroidal particles. The gelling agents which may be used in this process are hexamethylene tetraamine, urea or mixtures thereof. The gelling agents release ammonia at the elevated temperatures which sets or converts the hydrosol spheres into hydrogel spheres. The spheres are then continuously withdrawn from the oil bath and typically subjected to specific aging and drying treatments in oil and in ammoniacal solution to further improve their physical characteristics. The resulting aged and gelled particles are then washed and dried at a relatively low temperature of about 100° to 150°C and subjected to a calcination procedure at a temperature of about 450° to 700°C for a period of about 1 to 20 hours. The amount of phosphorus-containing alumina component present (as the oxide) in the catalyst can range from about 10 to 70 mass percent and preferably from about 20 to 50 mass percent.

The combined mixture preferably is dispersed into the oil bath in the form of droplets from a nozzle, orifice or rotating disk. Alternatively, the particles may be formed by spray-drying of the mixture at a temperature of from about 425° to 760°C. In any event, conditions and equipment should be selected to obtain small spherical particles; the particles preferably should have an average diameter of less than about .0 mm, more preferably from about 0.2 to 0.8 mm, and optimally from about 0.3 to 0.8 mm.

The degree of crystallinity of the aluminum phosphate binder can be controlled by varying the proportion of the phosphorus component. Material that is not in an amorphous phase generally is present as gamma-alumina; as the phosphorus content is decreased, the degree of crystallinity, therefore, is increased. The apparent bulk density of the spheres also varies with the phosphorus content, as a higher proportion of phosphorus decreases the average bulk density. Surface area also is controlled by phosphorus content: gamma-alumina oil-dropped spherical particles typically have surface areas up to about 250 $m^2$/g, while spheroidal particles of aluminum phosphate may have surface areas of up to about 450 $m^2$/g. Al/P atomic ratios of the binder/matrix generally range from about 1/10 to 100/1, and more typically from about 1/5 to 20/1.

The catalyst of the present invention may contain a halogen component. The halogen component may be either fluorine, chlorine, bromine or iodine or mixtures thereof, with chlorine being preferred. The halogen component is generally present in a combined state with the inorganic-oxide support. The optional halogen component is preferably well dispersed throughout the catalyst and may comprise from more than 0.2 to about 15 wt.%, calculated on an elemental basis, of the final catalyst. Preferably, however, the catalyst contains no added halogen other than that associated with other catalyst components.

The formed catalyst composite is dried at a temperature of from about 100° to about 320°C for a period of from about 2 to about 24 or more hours and, usually, calcined at a temperature of from 400° to about 650°C in an air atmosphere for a period of from about 0.1 to about 10 hours until the metallic compounds present are converted substantially to the oxide form.

The catalytic composite optimally is subjected to steaming to tailor its acid activity. The steaming may be effected at any stage of the zeolite treatment, but usually is carried out on the composite of zeolite and binder prior to incorporation of the platinum-group metal. Steaming conditions comprise a water concentration of about 5 to 100 volume-%, pressure of from about 100 kPa to 2 MPa, and temperature of from about 600° to about 1200°C; the steaming temperature preferably is at least about 650°C, more preferably at least about 750°C and optionally may be about 775°C or higher. In some cases, temperatures of about 800° to 850°C or more may be employed. The steaming should be carried out for a period of at least one hour, and periods of 6 to 48 hours are preferred.

Alternatively or in addition to the steaming, the composite may be washed with one or more of a solution of ammonium nitrate, a mineral acid, and/or water. Considering the first alternative, the catalyst may be washed with a solution of about 5 to 30 mass-% ammonium nitrate. When acid washing is employed, a mineral acid such as HCI or $HNO_3$ is preferred; sufficient acid is added to maintain a pH of from more than 1 to about 6, preferably from about 1.5 to 4. The catalyst is maintained in a bed over which the solution and/or water is circulated for a period of from about 0.5 to 48 hours, and preferably from about 1 to 24 hours. The washing may be effected at any stage of the preparation, and two or more stages of washing may be employed.

Prior to addition of the platinum-group metal component, the composite preferably is ion-exchanged with a salt solution containing at least one hydrogen-forming cation such as $NH_4$ or quaternary ammonium. The hydrogen-forming cation replaces principally alkali-metal cations to provide, after calcination, the hydrogen form of the zeolite component.

A platinum-group metal, including one or more of platinum, palladium, rhodium, ruthenium, osmium, and iridium, is an essential component of the present catalyst. The preferred platinum-group metals are platinum and palladium, with platinum being especially preferred. The platinum-group metal component may exist within the final catalyst composite as a compound such as an oxide, sulfide, halide, oxysulfide, etc., or as an elemental metal or in combination with one or more other ingredients of the catalyst composite. It is believed that the best results are obtained when substantially all of the platinum-group metal component exists in a reduced state. The platinum-group metal component generally comprises from about 100 to about 5000 mass-ppm (parts per million) of the final catalyst composite, calculated on an elemental basis, with a level of about 100 to about 2000 mass-ppm being particularly suitable. When using a platinum component, very low levels of about 200 to 800 mass-ppm of platinum on the catalyst, on an elemental basis, are favored; levels of less than about 600 mass-ppm are especially favored and levels of about 300 to about 500 mass-ppm show excellent results. When using a palladium component, levels of about 400 to 2000 mass-ppm of palladium on the catalyst, on an elemental basis, are favored and levels of from about 500 to about 1200 mass-ppm are especially favored.

The platinum-group metal component may be incorporated into the catalyst composite in any suitable manner. One method of preparing the catalyst involves the utilization of a water-soluble, decomposable compound of a platinum-group metal to impregnate the calcined sieve/binder composite. Alternatively, a platinum-group metal compound may be added at the time of compositing the sieve component and binder. Yet another method of effecting a suitable metal distribution is by compositing the metal component with the binder prior to co-extruding the sieve and binder. Complexes of platinum-group metals which may be employed according to the above or other known methods include chloroplatinic acid, chloropalladic acid, ammonium chloroplatinate, bromoplatinic acid, platinum trichloride, platinum tetrachloride hydrate, platinum dichlorocarbonyl dichloride, tetraamineplatinum chloride, dinitrodiaminoplatinum, sodium tetranitroplatinate (II), palladium chloride, palladium nitrate, palladium sulfate, diaminepalladium (II) hydroxide, tetraaminepalladium (II) chloride, and the like. It is within the scope of the present invention that the catalyst may contain other metal components known to modify the effect of the platinum-group metal component.

After addition of the metal component, the resultant catalytic composite usually is dried at a temperature of about 100° to about 320°C for a period of from about 1 to about 24 or more hours. The dried composite then is calcined at a temperature of from about 400° to about 600°C in an air atmosphere for a period of from about 0.1 to 10 hours to convert the metallic components substantially to the oxide form.

The calcined composite optimally is subjected to a substantially water-free reduction step to insure a uniform and finely divided dispersion of the optional metallic components. The reduction optionally may be effected on the catalyst as loaded in the isomerization-process reactor of the present invention prior to the startup of the isomerization process. Substantially pure and dry hydrogen (i.e., less than 20 vol. ppm $H_2O$) preferably is used as the reducing agent in this step. The reducing agent contacts the catalyst at conditions, including a temperature of from about 200° to about 650°C and for a period of from about 0.5 to about 10 hours, effective to reduce substantially all of the Group VIII metal component to the metallic state. In some cases the resulting reduced catalyst composite may also be beneficially subjected to presulfiding by a method known in the art to incorporate in the catalyst composite from about 0.01 to about 0.5 mass-% sulfur, on an elemental basis, into the catalyst.

The finished catalyst has a McBain water capacity at $6.13 \times 10^2$ Pa (4.6 torr) of less than 7 mass%, more particularly between 2 and 7 mass-%, preferably between 3.0 and 5.0 mass-%, and with the usual optimum being between 3.5 and 4.5 mass-%. Water capacity may be measured by the following procedure:

*Activation step:*

Empty pails readings *(W1)* are taken, then the catalyst is activated in vacuum for about 16 hours overnight at 375° to 400°C; the vacuum of the system should be less than 1 millitorr. The catalyst is cooled to room temperature and the McBain tubes containing the activated catalyst are closed off; a room-temperature reading (*W2*) of the activated catalyst then is taken. An ice bath then is assembled around the water reservoir so that the water is maintained at 0°C during the adsorption step

*Adsorption step:*

The manifold is purged of water vapor by opening the water reservoir; the mercury U-type manometer should read a pressure of $6.13 \times 10^2$ Pa (4.6 torr). The tubes are opened and the adsorption starting time recorded. The catalyst is allowed to equilibrate for at least 1.5 hours, the final time is recorded, and the reading *(W3)* is taken of the catalyst equilibrated with water vapor. The water capacity is calculated as:

$$\text{Capacity, mass-\%} = [(W2-W3)/(W1-W2)]*100$$

The gradual accumulation of coke and other deactivating carbonaceous deposits on the catalyst during the operation of the isomerization process will eventually reduce the activity and selectivity of the process to a level such that regeneration is desirable. When the performance of the catalyst has decayed to the point where it is desired to regenerate the catalyst, the introduction of the hydrocarbon charge stock into the conversion zone containing the catalyst is stopped and the conversion zone purged with a suitable gas stream. Regeneration may be performed to restore catalyst activity and selectivity, either in situ or by unloading and regenerating the catalyst in an off-line facility.

EXAMPLES

Example I

An aluminum-phosphate-bound MFI catalyst was prepared to illustrate the transalkylation process of the invention. A first solution was prepared by adding phosphoric acid to an aqueous solution of hexamethylenetetraamine (HMT) in an amount to yield a phosphorus content of the finished catalyst equal to about 11 mass-%. A second solution was prepared by adding an ammonia-exchanged MFI-type zeolite having an $Si/Al_2$ ratio of about 39 to enough alumina sol, prepared by digesting metallic aluminum in hydrochloric acid, to yield a zeolite content in the finished catalyst equal to about 67 mass-%. These two solutions were commingled to achieve a homogeneous admixture of HMT, phosphorus, alumina sol, and zeolite. This admixture was dispersed as droplets into an oil bath maintained at about 93°C. The droplets remained in the oil bath until they set and formed hydrogel spheres having a diameter of about 1.6 mm. The spheres were removed from the oil bath, water washed, air dried, and calcined at a temperature of about 550°C. The calcined spheres then were subjected to steaming at a temperature of about 660°C in an atmosphere of 40% steam in air for 12 hours.

The steamed spheres then were metal-impregnated using a solution of tetraamine platinum chloride. Upon completion of the impregnation, the catalyst was dried, oxidized, and reduced to yield a catalyst containing about 460 mass parts per million (ppm) platinum. This isomerization catalyst, utilized to illustrate the process of the invention, was designated as Catalyst A.

Example II

A silica-bound MFI catalyst was prepared as a control. Ammonia-exchanged MFI-type zeolite having an $Si/Al_2$ ratio of about 38 was blended with Methocel in a muller. Hydrated amorphous silica powder, Ludox AS-40, was added in proportion to effect a final catalyst MFI content of about 67 mass-%. The mixture and sufficient deionized water to effect an extrudable dough having a moisture content of about 40 mass-% was blended thoroughly in the muller. The dough then was extruded through a cylindrical die to form cylindrical extrudates having a diameter of about 1.6 mm. The extrudates then were water washed, air dried, and calcined at a temperature of about 550°C and then subjected to steaming at a temperature of about 660°C in an atmosphere of 40% steam in air for 12 hours.

The steamed extrudates then were column-washed with ammonium nitrate at 88°C for 5 hours, then washed with deionized water and dried at 510°C for about 9 hours. The dried extrudates were metal-impregnated by rolling in a solution of chloroplatinic acid at temperatures of 60-100°C for 6-7 hours. Upon completion of the impregnation, the composite was dried, oxidized, and reduced to yield a catalyst containing about 460 mass parts per million (ppm) platinum. This isomerization catalyst was designated as Catalyst A'.

Example III

An alumina-bound catalyst was prepared as a control for comparison of isomerization results with Catalyst A. A first solution of aluminum nitrate was prepared to which was added a second solution of $NH_4OH$. The second solution was added slowly maintaining the pH of the first solution at 8 until gellation of the aluminum occurred. The hydrogel of alumina was slurried with water and filtered to obtain a filter cake having a moisture content of approximately 90 wt.%. A portion of this filter cake was dried at 150°C to a moisture content of about 60 wt. %. This dried material was then ground to produce powdered alumina.

An extrudable dough was prepared by combining and mixing about 67 mass-% MFI zeolite with hydrogel alumina and powdered alumina. The dough then was extruded to form cylindrical extrudates. The extrudates were directly subjected to calcination in flowing air at a temperature of about 600°C.

The calcined extrudates were then subjected to an impregnation procedure using a solution of tetraamine platinum chloride. The catalyst then was dried, oxidized, and reduced to yield a catalyst containing about 400 mass-ppm platinum. This reference catalyst is designated Catalyst X.

Example IV

A further control catalyst was prepared for comparison with the catalyst of the invention. This catalyst consisted essentially of approximately 11 mass-% hydrogen-form ZSM-5 zeolite and 0.29 mass-% platinum, with the remainder being alumina binder. MFI zeolite was added to an alumina sol solution, prepared by digesting metallic aluminum in hydrochloric acid, in an amount sufficient to yield a zeolite content in the finished catalyst of about 11 mass-%. A second solution of hexamethylenetetramine (HMT) was prepared and added to the zeolite/alumina sol mixture to give homogeneous admixture. This admixture was then dispersed as droplets into an oil bath maintained at about 93°C. The droplets remained in the oil bath at 150°C until they set and formed hydrogel spheres. These spheres were removed from the oil bath, water washed with a 0.5% ammonia/water solution, air dried, and calcined at a temperature of about 650°C. These calcined spheres were then coimpregnated with a solution of chloroplatinic acid, lead as $Pb(NO_3)_2$ and hydrochloric acid to yield about 0.2 mass-% platinum and 0.8 mass-% lead on the finished catalyst. The impregnated spheres were oxidized, subjected to a reducing environment of $H_2$ at 565°C, and sulfided with $H_2S$. This catalyst of the known art was designated Catalyst Y.

Example V

The comparative isomerization performance of the catalyst of the invention and the control catalysts were evaluated using a pilot plant processing non-equilibrium $C_8$-aromatic feed having the following composition in mol-%:

| ethylbenzene | 7.3-7.4% |
|---|---|
| para-xylene | 0.1% |
| meta-xylene | 70.8-71.3% |
| ortho-xylene | 21.3-21.8% |

This feed was isomerized at a pressure of 1.3 MPa, a mass hourly space velocity of 10 $hr^{-1}$, and a hydrogen/hydrocarbon mole ratio of 4. Reactor temperature was adjusted to effect a range of conversion levels, expressed as the disappearance per pass of ethylbenzene. Ethylbenzene, plus the small amount of xylene loss, is converted primarily to benzene and toluene with smaller amounts of light gases. Results of the tests, comparing the loss of valuable xylenes, may be summarized as follows at 65% ethylbenzene conversion:

| Catalyst: | A | A' | X | Y |
|---|---|---|---|---|
| Temperature, °C | 391 | 364 | 372 | 391 |
| Product para-xylene/xylenes, % | 23.9 | 23.7 | 23.8 | 24.0 |
| Xylenes loss, % | 1.3 | 2.6 | 7.4 | 2.6 |

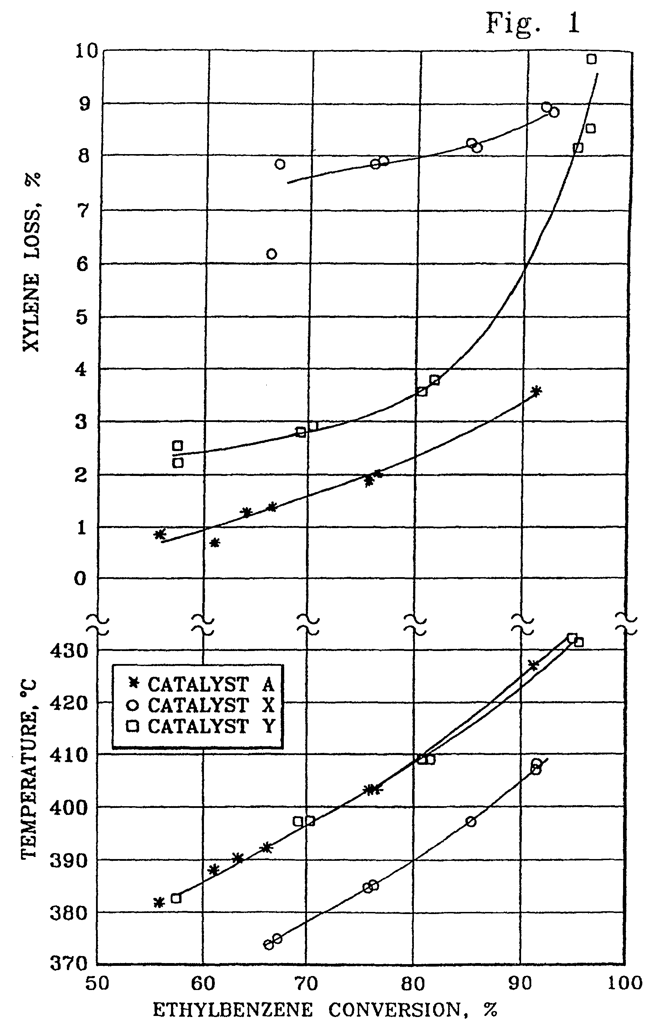

Catalyst A of the invention showed substantially lower xylene losses at consistent isomerization conditions than the catalysts of the known art. Catalyst A' (control) demonstrated exceptional activity as evidenced by the low temperature required to achieve 65% ethylbenzene conversion, along with low xylene losses in comparison to catalysts of the prior art. The relative performance of the catalysts A, X and Y, indicating xylene losses and activity as a function of conversion, is shown in Figure 1.

Example VI

A series of catalysts of the invention were prepared to compare the effect of the concentration of platinum-group metal on performance in an isomerization process. Calcined spheres having a zeolite content of about 67 mass-% were prepared and steamed according to the procedure described in Example I. The spheres then were impregnated using a solution of tetraamine platinum chloride, dried, oxidized and reduced. The spheres were designated and had platinum contents as follows in mass parts per million:

| Catalyst | Platinum |
|----------|----------|
| A | 460 ppm |
| B | 200 ppm |
| C | 390 ppm |
| D | 540 ppm |
| E | 740 ppm |

Example VII

The comparative isomerization performance of the catalysts of the invention having varying platinum contents was evaluated using a pilot plant processing non-equilibrium $C_8$-aromatic feed having the following composition in mol-%:

| ethylbenzene | 7.3% |
|--------------|------|
| para-xylene | 0.1% |
| meta-xylene | 71.3% |
| ortho-xylene | 21.3% |

This feed was isomerized at a pressure of a pressure of 1.3 MPa, a mass hourly space velocity of 10 hr$^{-1}$, and a hydrogen/hydrocarbon mole ratio of 4. The ratios of para-xylene/xylenes was established at about 23.9%. Reactor temperature was adjusted as shown to effect a range of conversion levels, expressed as the disappearance per pass of ethylbenzene. Results of the tests with respect to loss of valuable xylenes may be summarized as follows at 65% ethylbenzene conversion:

| Catalyst: | Temperature, °C | Xylene Loss |
|-----------|-----------------|-------------|
| A | 392 | 1.5% |
| B | 408 | 1.4% |
| C | 382 | 1.7% |
| D | 392 | 3.8% |
| E | 388 | 4.7% |

Xylene loss was minimized at catalyst platinum contents of about 400 mass-ppm and less. Catalyst activity was more favorable at catalyst platinum contents of about 400 mass-ppm and above.

Example VIII

The effect of steaming temperature on catalyst properties and performance was studied by preparing and testing a series of catalysts. Calcined spheres having a zeolite content of about 67 mass-% were prepared and steamed according to the procedure described in Example I. Steaming was effected either by ramping to the temperature indicated ("ramp") or by immediately subjecting the catalyst to the temperature ("flat"). The spheres then were impregnated to effect a platinum content of the catalyst of 350-460 mass-ppm, dried, oxidized and reduced. The finished catalysts

had $6{\cdot}13 \times 10^2$ Pa (4.6 torr) water capacities in mass-%, as measured by the previously described McBain water-capacity test, as indicated below:

| Catalyst | Steaming Temp., °C | Water Capacity |
|----------|--------------------|----------------|
| A | 660 ramp | 5.02 |
| F | 705 ramp | 6.01 |
| G | 732 ramp | 5.44 |
| H | 760 ramp | 5.26 |
| J | 788 ramp | 5.36 |
| K | 788 flat | 4.29 |

Example IX

The comparative isomerization performance of the catalysts of Example VII was evaluated using a pilot plant processing non-equilibrium $C_8$-aromatic feed as described in Example IV. This feed was isomerized at a pressure of a pressure of 1.3 MPa, a mass hourly space velocity of 10 $hr^{-1}$, a hydrogen/hydrocarbon mole ratio of 4 and a temperature as indicated below. Results of the tests may be summarized as follows at 65% ethylbenzene conversion:

| Catalyst | Temperature, °C | Xylene Loss |
|----------|-----------------|-------------|
| A | 392 | 1.5% |
| F | 357 | 2.7% |
| G | 363 | 2.5% |
| H | 363 | 2.0% |
| J | 357 | 3.3% |
| K | 382 | 1.4% |

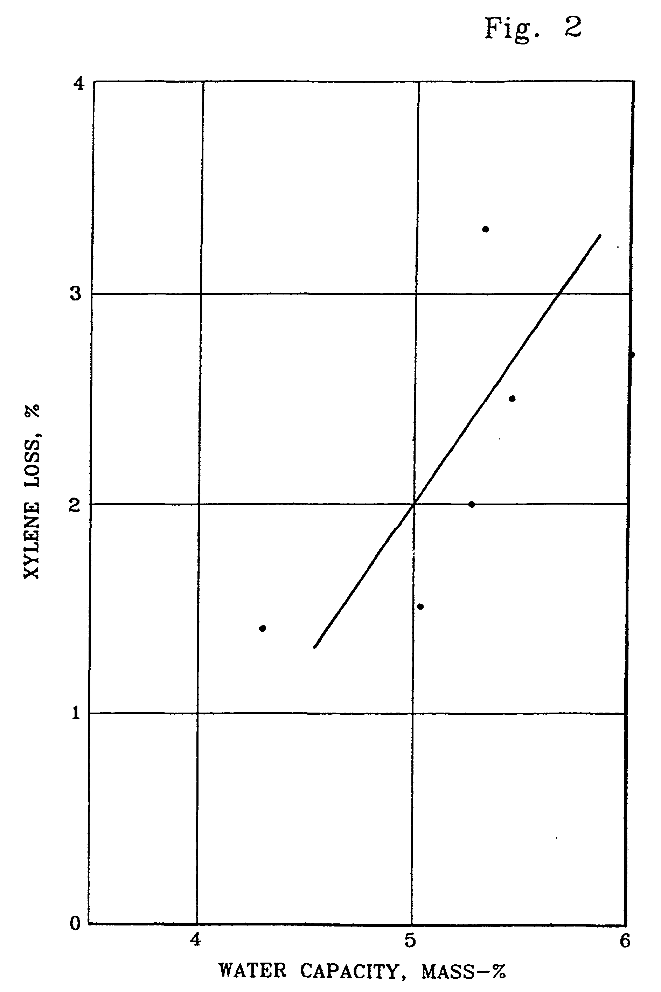

Reduced water capacity is a concomitant of catalyst activity, and xylene loss is low when this is achieved with high-temperature steaming without ramping. The results are shown graphically in Figure 2.

Example X

A further series of catalysts was prepared employing high steaming temperatures according to the invention, and performance was studied according to previously described testing procedures. Calcined spheres having a zeolite content of about 67 mass-% were prepared and steamed according to the procedure described in Example I. Steaming was effected in successive zones at temperatures of about 725° and 850°C respectively. Catalysts O and Q were ammonium-exchanged. The spheres then were impregnated to effect a platinum content of the catalyst of 350-460 mass-ppm, dried, oxidized and reduced. The finished catalysts had $6{\cdot}13 \times 10^2$ Pa (4.6 torr) water capacities in mass-%, as measured by the previously described McBain water-capacity test, as indicated below: The comparative isomerization performance of the catalysts was evaluated using a pilot plant processing non-equilibrium $C_8$-aromatic feed as described in Example IV. This feed was isomerized at a pressure of a pressure of 1.3 MPa, a mass hourly space velocity of 10 $hr^{-1}$, a hydrogen/hydrocarbon mole ratio of 4 and a temperature as indicated below. Results of the tests were as follows:

| Catalyst | Water Capacity | Temp., °C | Ethylbenzene Conversion | Xylene Loss |
|----------|----------------|-----------|-------------------------|-------------|
| L | 3.75 | 404 | 61.7% | 2.1% |
| M | 4.1 | 415 | 61.9% | 1.15% |
| N | 4.2 | 403 | 61.1% | 1.9% |
| O | 4.2 | 391 | 62.6% | 1.6% |
| P | 4.15 | 404 | 65.4% | 1.6% |
| Q | 4.15 | 393 | 67.2% | 1.3% |

Example XI

Palladium-containing catalysts of the invention were prepared in the manner described in Example I, except that the steamed spheres were impregnated with tetraamine palladium chloride. The finished catalysts had the following palladium contents in mass-ppm on an elemental basis:

| Catalyst L | 540 |
|---|---|
| Catalyst M | 1130 |

Example XII

The isomerization performance of the palladium-containing catalysts of the invention was evaluated using a pilot plant processing non-equilibrium $C_8$-aromatic feed having the following composition in mol-%:

| ethylbenzene | 7.3% |
|---|---|
| para-xylene | 0.1% |
| meta-xylene | 71.4% |
| ortho-xylene | 21.2% |

This feed was isomerized at a pressure of 1.3 MPa, a mass hourly space velocity of 10 hr$^{-1}$, and a hydrogen/hydrocarbon mole ratio of 4. Reactor temperature was adjusted to effect a range of conversion levels, expressed as the disappearance per pass of ethylbenzene. Ethylbenzene, plus the small amount of xylene loss, is converted primarily to benzene and toluene with smaller amounts of light gases. Results of the tests may be summarized as follows at 65% ethylbenzene conversion:

| Catalyst: | L | M |
|---|---|---|
| Temperature, °C | 385 | 382 |
| Product para-xylene/xylenes, % | 23.9 | 23.9 |
| Xylenes loss, % | 1.4 | 1.5 |

**Claims**

1.  A process for the isomerization of a non-equilibrium feed mixture of xylenes and ethylbenzene comprising contacting the feed mixture in the presence of hydrogen with a catalyst comprising a zeolitic aluminosilicate having a pore diameter of from 5 to 8 A said finished catalyst having a 6.3 x 10$^2$ Pa (4.6 torr) water capacity of less than 7 mass-%, a platinum-group metal component and contains an amorphous aluminum phosphate binder in an isomerization zone at isomerization conditions comprising a temperature of from 100° to 600°C, a pressure of from 100 kPa to 10 Mpa, a mass hourly space velocity of from 0.5 to 100 hr$^{-1}$ to obtain an isomerized product comprising a higher proportion of at least one xylene isomer than in the feed mixture wherein the catalyst is in the form of oil dropped spheres.

2.  The process of Claim 1 wherein the xylene isomer is para-xylene.

3.  The process of Claims 1or 2 wherein the isomerization conditions comprise a temperature of from 350° to 500°C, a pressure of from 100 kPa to 5 Mpa absolute, and a mass hourly space velocity of from 2 to 50 hr$^{-1}$.

4.  The process of Claims 1, 2 or 3 wherein the zeolitic aluminosilicate comprises a pentasil zeolite selected from the group consisting of MFI, MEL, MTW and TON.

5.  The process of Claims 1, 2, 3 or 4 wherein the finished catalyst has a 6·13 x 10$^2$ Pa (4.6 torr) water capacity of 3-5 mass-% or less.

6.  The process of Claim 1 wherein the platinum-group metal component comprises from 200 to 800 mass-ppm platinum on an elemental basis.

7.  The process of Claim 1 further comprising recovering ortho-xylene from one or both of the isomerized product and a fresh $C_8$-aromatics feed.

**Patentansprüche**

1. Verfahren für das Isomerisieren einer sich nicht im Gleichgewicht befindenden Einspeisemischung von Xylolen und Ethylbenzol, welches Verfahren das Kontaktieren der Einspeisemischung in Gegenwart von Wasserstoff mit einem Katalysator umfasst, der ein Zeolith-Aluminosilikat mit einem Porendurchmesser von 5 bis 8 Å umfasst, wobei der fertige Katalysator eine 6,3 x $10^2$ Pa- (4,6 Torr-) Wasserkapazität von weniger als 7 Massen-%, eine Metallkomponente der Platingruppe aufweist und ein amorphes Aluminiumphosphatbindemittel enthält, in einer Isomerisierungszone unter Isomerisierungsbedingungen, die eine Temperatur von 100 bis 600 °C, einen Druck von 100 kPa bis 10 MPa, eine Massenstundenraumgeschwindigkeit von 0,5 bis 100 h$^{-1}$ umfassen, zur Erzielung eines isomerisierten Produkts, das einen erhöhten Anteil mindestens eines Xylolisomers umfasst als die Einspeisemischung, wobei der Katalysator in Form von tropfenförmig in Öl gebildeten Kugeln vorliegt.

2. Verfahren nach Anspruch 1, wobei es sich bei dem Xylolisomer um Para-Xylol handelt.

3. Verfahren nach Anspruch 1 oder 2, wobei die Isomerisierungsbedingungen eine Temperatur von 350 bis 500 °C, einen absoluten Druck von 100 kPa bis 5 MPa und eine Massenstundenraumgeschwindigkeit von 2 bis 50 h$^{-1}$ umfassen.

4. Verfahren nach Anspruch 1, 2 oder 3, wobei das Zeolith-Aluminosilikat einen Pentasilzeolith umfasst, der aus der Gruppe ausgewählt wird, die aus MFI, MEL, MTW und TON besteht.

5. Verfahren nach Anspruch 1, 2, 3 oder 4, wobei der fertige Katalysator eine 6,3 x $10^2$ Pa- (4,6 Torr-) Wasserkapazität von 3 - 5 Massen-% oder weniger aufweist.

6. Verfahren nach Anspruch 1, wobei die Metallkomponente der Platingruppe 200 bis 800 Massen-ppm Platin auf Elementbasis umfasst.

7. Verfahren nach Anspruch 1, das des Weiteren das Gewinnen von Ortho-Xylol aus einem oder beiden der isomerisierten Produkte und einer frischen Einspeisung von aromatischen C$_8$-Verbindungen umfasst.

**Revendications**

1. Procédé d'isomérisation d'un mélange d'alimentation non équilibré de xylènes et d'éthylbenzène comprenant la mise en contact du mélange d'alimentation en présence d'hydrogène avec un catalyseur comprenant un aluminosilicate de zéolite ayant un diamètre de pores allant de 5 à 8 Angstroems, ledit catalyseur fini ayant une capacité d'eau à 6,3 x $10^2$ Pa (4,6 Torrs) de moins de 7% en masse, un composant métallique du groupe du platine et contenant un liant de phosphate d'alumine amorphe dans une zone d'isomérisation à des conditions d'isomérisation, comprenant une température allant de 100°C à 600°C, une pression allant de 100 kPa à 10 MPa, une vitesse spatiale horaire massique allant de 0,5 à 100 hr$^{-1}$ pour obtenir un produit d'isomérisation comprenant une proportion plus élevée d'au moins un isomère du xylène que dans le mélange d'alimentation, dans lequel le catalyseur est sous la forme de sphères de gouttes d'huile.

2. Procédé selon la revendication 1, dans lequel l'isomère du xylène est le para-xylène.

3. Procédé selon la revendication 1 ou la revendication 2, dans lequel les conditions d'isomérisation comprennent une température allant de 350°C à 500°C, une pression allant de 100 kPa à 5 MPa en pression absolue, et une vitesse spatiale horaire massique allant de 2 à 50 hr$^{-1}$.

4. Procédé selon les revendications 1, 2 ou 3, dans lequel l'aluminosilicate de zéolite comprend un zéolite de pentasil sélectionné parmi le groupe constitué de MFI, de MEL, de MTW et de TON.

5. Procédé selon les revendications 1, 2, 3 ou 4, dans lequel le catalyseur fini possède une capacité d'eau à 6,3 x $10^2$ Pa (4,6 Torrs) de 3-5 % en masse ou moins.

6. Procédé selon la revendication 1, dans lequel le composant de métal du groupe du platine comprend de 200 à 800 ppm en masse de platine sur une base élémentaire.

7. Procédé selon la revendication 1, comprenant en outre la récupération de l'ortho-xylène de l'un ou des deux du produit isomérisé et d'une alimentation fraîche en aromatiques en $C_8$.

Fig. 1

Fig. 2